# NOUVEAU FASCICULE DE BREVET EUROPEEN

(11) **EP 1 440 092 B2**
(45) Date de publication et mention de la décision concernant l'opposition: **17.12.2014**
(45) Mention de la délivrance du brevet: 18.01.2012
(21) Numéro de dépôt: 02796824.7
(22) Date de dépôt: 23.10.2002
(51) Int. Cl.: A61K 8/64, A61Q 5/00, A61Q 17/00

(54) **COMPOSITION PHOTOACTIVABLE ET UTILISATIONS**
PHOTOAKTIVIERBARE ZUSAMMENSETZUNG UND IHRE VERWENDUNG
PHOTOACTIVATABLE COMPOSITION AND THE USE OF SAME

(30) Priorité: 29.10.2001 FR 0113970
(43) Date de publication de la demande: 28.07.2004
(73) Titulaire: L'Oréal, 75008 Paris (FR)
(72) Inventeur: VIC, Gabin, F-60280 Venette (FR); LIVOREIL, Aude, F-75006 Paris (FR)
(74) Mandataire: Casalonga, Axel
(86) Numéro de dépôt international: PCT/FR2002/003632
(87) Numéro de publication internationale: WO 2003/037830

(56) Documents cités:
- WO-A2-01/06829
- FR-A- 2 605 220
- US-A- 4 695 285

## Description

La présente invention décrit un composé photo-activable comprenant une molécule-support choisie parmi les composés non-polymériques carbonés et les polymères tels que définis ultérieurement, sur laquelle sont liés de manière covalente au moins deux groupes fonctionnels photo-activables particuliers.

L'invention concerne en particulier les compositions cosmétiques contenant au moins un agent cosmétique particulier et de tel(s) composé(s) photo-activable(s), le(s)dit(s) composé(s) photoactivable(s) étant lié(s) de manière covalente à l'agent ou lesdits agents cosmétiques, et les procédés de traitement cosmétique mettant en oeuvre de telles compositions.

Les produits généralement mis en oeuvre pour fixer des agents cosmétiques sur les matières kératiniques présentent l'inconvénient d'être éliminés très vite au lavage ou lors d'autres traitements.

Aussi les inventeurs de la présente demande ont cherché une composition permettant de fixer, de manière durable sur les matières kératiniques des agents cosmétiques.

Il est connu dans l'art antérieur des procédés de coloration utilisant en tant qu'agent colorant des composés photo-activables, ainsi le procédé de coloration des matières kératiniques décrit dans la demande de brevet FR 2 605 220 consiste à mettre les matières kératiniques en contact avec un azoture aromatique ou un azidoindole puis à exposer lesdites matières kératiniques à une source de lumière adéquate pour développer la couleur.

Il est aussi connu de la demande de brevet EP 1152013 des produits comprenant des composés dérivés de chitosan ou de chitine comportant au moins une fonction photo-activable.

On connaît également, dans l'art antérieur, des procédés pour réaliser des dépôts de matières sur des fibres capillaires. Ces procédés nécessitent la présence de groupes réactifs sur la matière à déposer qui puissent réagir avec des groupes réactifs des fibres capillaires. Il s'agit de procédés multi-étapes au cours desquels on modifie la matière à déposer d'une part et les fibres capillaires d'autre part afin que des sites activés de part et d'autre puissent interagir. On peut citer à cet égard, le brevet US 5 211 942.

Le but de la présente demande est de proposer des compositions qui permettent, de préférence en une seule étape, de fixer de manière durable un agent cosmétique sur les matières kératiniques. Ces compositions comprennent des composés qui assurent la liaison entre l'agent cosmétique et les matières kératiniques et sont fixés à l'agent cosmétique et aux matières kératiniques par greffage covalent, soit simultanément, soit séquentiellement.

La présente invention décrit un composé photo-activable comprenant une molécule-support, avec ou sans activité cosmétique, choisie parmi les composés non-polymériques carbonés et, les polymères tels que definis ultérieurement et sur laquelle sont liés de façon covalente au moins deux entités (groupes fonctionnels photo-activables) particulières contenant des groupes chimiques qui, après irradiation entre 200 et 450 nm, sont transformés en espèces réactives capables de réagir avec des groupes chimiques appartenant à d'autres molécules. .

En particulier, l'invention a pour objet une composition cosmétique comprenant au moins un composé photo-activable selon l'invention tel que défini à la revendication 1 et au moins un actif cosmétique particulier tel que défini à la revendication 1,le composé photoactivable et l'actif cosmétique étant liés de manière covalente. .

Les compositions cosmétiques selon la présente invention comprennent un solvant cosmétiquement acceptable tel que l'eau, l'éthanol ou leurs mélanges. Ce solvant pourra également contenir d'autres solvants organiques tels que les alcanes en C₅ à C₁₀, l'acétone, la méthyléthylcétone, l'acétate de méthyle, l'acétate d'éthyle, l'acétate de butyle, le diméthoxyéthàne, le diéthoxyéthane et leurs mélange

Le composé photo-activable comprend une molécule support à activité cosmétique.

Ladite composition cosmétique peut être utilisée en tant qu'agent de coloration de la peau , des ongles ou des cheveux, en tant qu'agent hydratant, en tant qu'agent destiné à augmenter la brillance notamment des cheveux (agent brillant), en tant que filtre solaire, en tant qu'agent de conditionnement, en tant qu'agent de mise en forme des fibres kératiniques:

Un autre objet de l'invention comprend un procédé de traitement cosmétique des matières kératiniques, préférentiellement des cheveux, qui consiste à appliquer sur ces matières kératiniques une composition cosmétique selon l'invention en une étape puis à exposer lesdites matières kératiniques à un rayonnement d'une ou plusieurs longueurs d'ondes comprises entre 200 et 450 nm, de préférence 200 et 400 nm et de manière encore plus préférée 250 et 400 nm. -

Par matières kératiniques, on entend les cheveux, cils, sourcils, poils, ongles, peau.

En tant que molécule-support utilisable pour la préparation du composé photoactivable selon l'invention, on utilise les composés non-polymériques carbonés et/ou , les polymères.

Par composé non-polymérique carboné, on entend une chaîne linéaire ou ramifiée ou cyclique, contenant de 1 à 80 atomes de carbone, de préférence 1 à 50 atomes de carbone, comprenant de manière éventuelle 1 à 25, de préférence 1 à 10 héteroatomes (O, N, Si, S, P), cette chaîne étant éventuellement substituée par 1 à 60 substituants choisis parmi les groupes hydroxyle, amine, thiol, carbamate, éther, acide, ester, amide, cyano, uréido, halogène (en particulier fluor ou chlore).

Par polymère, on entend tous les polymères utilisables en cosmétique, naturels ou synthétiques et en particulier les polymères obtenus par polymérisation radicalaire ou par polycondensation ou par ouverture de cycles. Ces polymères peuvent être linéaires, ramifiés, en étoile.

En tant que polymères naturels, on utilisera de préférence les polysaccharides (par exemple les dextrans, celluloses, amidons, chitosane, pullulane, insuline, carraghénane, guar, alginates, xanthanes, acide hyaluronique), les protéines telles que l'albumine, l'ovalbumine, la kératine, le collagène.

Les polymères naturels peuvent être modifiés chimiquement, on pourra ainsi introduire dans la chaîne principale de ce polymère naturel au moins un groupe choisi parmi les hydroxyalkyle, carboxyalkyle, amino, thio, des fonctions aldéhyde, époxy.

Les polymères synthétiques peuvent être des homopolymères ou des copolymères.

De préférence on utilisera les polyuréthannes, polyurées, polyéthers, polyesters, polyamides.

Il pourra également s'agir des polymères dendritiques ou dendrimères tels que décrits par D.A.Tomalia et al, Angewandlte Chemie, Int.Engl. Ed.,vol.29, n°2 p 138-175. Ces dendrimères sont des structures moléculaires construites autour d'un motif central généralement polyvalent. Autour de ce motif central, des motifs ramifiés d'allongement de chaîne sont enchaînés en couches concentriques et selon une structure parfaitement déterminées donnant ainsi naissance à des macromolécules symétriques, monodispersées ayant une structure chimique et stéréochimique bien définie.

Il pourra également s'agir de polymères dendritiques de type polymères hyperramifiés. Des exemples de ces polymères sont décrits dans les demandes de brevet WO 93 17060et WO 96 12754. La partie de ces demandes de brevet relative à ces polymères hyperramifiés et à leur synthèse est incorporée ici par référence.

Il pourra également s'agir de dendrons tels que définis dans l'article de D.A.Tomalia.

Au moins deux des substituants de ladite molécule-support doivent être susceptibles de réagir avec des groupes fonctionnels photo-activables pour donner le composé photo-activable selon la présente demande.

En tant que groupes fonctionnels photo-activables, on entend les diazirines tels que définis dans la revendication 1. Ces groupes fonctionnels photo-activables peuvent générer des espèces réactives (carbènes, nitrènes, espèces radicalaires...) par simple exposition à un rayonnement d'une ou plusieurs longueurs d'ondes comprises entre 200 et 450 nm, de préférence 200 et 400 nm et de manière encore plus préférée 250 et 400 nm. Cette exposition peut être réalisée via des simulateurs solaires, des lampes UV ou par simple action du rayonnement solaire. Ces espèces réactives présentent la propriété de pouvoir s'insérer de façon non sélective dans de nombreuses liaisons chimiques telles que des liaisons C-H, N-H, O-H, C-C, C=C, S-H, C≡C de la matière kératinique ou de l'agent cosmétique.

Les diazirines utilisables au sens de la présente demande sont des molécules ayant pour formule : 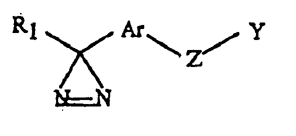 dans laquelle R₁ est choisi dans le groupe formé par un atome d'hydrogène, un radical alkyle en C₁ à C₁₀ linéaire ou ramifié, un radical alkényle en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', C≡N, COOH, F, Cl, Br, I, OR, COOR', SO₃H, COR', SH, SR', OH, où R' est un radical alkyle en C₁ à C₁₀.

Z est une simple liaison ou un groupe espaceur qui est une chaîne carbonée, linéaire ramifiée ou cyclique, saturée ou insaturée, en C₁-C₁₀₀, de préférence C₁-C₅₀, cette chaîne pouvant être interrompue par des hétéroatomes tels que le souffre, l'oxygène, l'azote, le silicium ou le phosphore. Elle peut aussi comprendre un ou plusieurs substituants tels que des groupes hydroxyles, amines, thiols, carbamates, éthers, acides, esters, amides, cyano, uréido. De manière préférée, il s'agira d'un polyol ou d'un polyalkylèneglycol (PEG ou PPG).

Y est la fonction qui permet d'établir la liaison covalente entre la molécule support et l'entité comportant le ou les groupes fonctionnels photoactivables.

Y représente une fonction choisie dans le groupe formé par les alcools, amines, thiols, thiosulfates, acides carboxyliques et ses dérivés tels que les anhydrides, chlorures d'acide, esters, les acétals et hémi-acétals, les aminals et hémi-aminals, les cétones, les aldéhydes, alpha-hydroxycétones, les alpha-halocétones époxydes, les lactones, thiolactones, azalactones, isocyanate, thiocyanate, imines, imides (succinimides, glutimides), imidoesters, aziridines, imidates, oxazine et oxazoline, oxazinium et oxazolinium, les halogénes (fluor, chlore, iode, brome), les chlorotriazines, chloropyrimidines, chloroquinoxalines, chlorobenzotriazoles, les halogénures (X= F, Cl, I ou Br) de sulfonyle : SO₂X, les siloxanes, silanols, silanes, les pyridyldithio, les N-hydroxysuccinimide esters, les vinyles activés ou non activés parmi lesquels les acrylonitriles, esters acryliques et méthacryliques, acides et esters crotoniques, acides et esters cinnamiques, styrènes, butadiènes, éthers de vinyle, vinyle cétone, esters maléiques, maléimides, vinyl sulfones, les hydrazines, les phenyls glyoxals.

Ar représente un noyau aromatique choisi dans le groupe formé par :

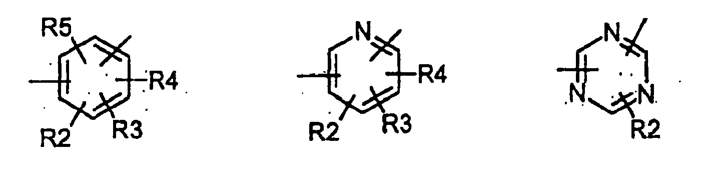

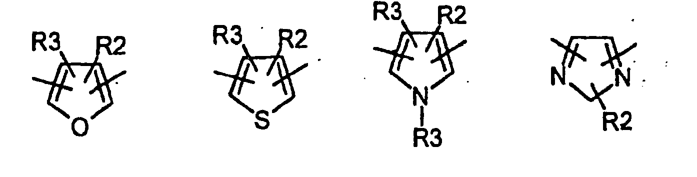

Dans lesquels R₂, R₃, R₄, et R₅ représentent, indépendamment l'un de l'autre, des radicaux choisis dans le groupe formé par un atome d'hydrogène, un radical alkyle en C₁ à C₁₀ linéaire ou ramifié, un alkylène en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', C≡N, COOH, F, Cl, Br, I, OR', COOR', COR', SH, SR', OH, SO₃H où R' est un radical alkyle en C₁ à C₁₀.

Les réactions chimiques qui seront mises en oeuvre pour synthétiser le composé photo-activable déprendront des groupements Y et des groupements complémentaires sur la molécule-support, toutes les réactions chimiques classiques pouvant être utilisées. De façon analogue, on pourra prévoir de protéger les groupements que l'on ne souhaite pas voir réagir. Toutes les réactions classiques de protection et de déprotection des fonctions réactives peuvent être utilisées.

Au moyen des composés photo-activables selon l'invention, on assure le greffage covalent des actifs cosmétiques présents dans le milieu sur la matière kératinique.

Le greffage du composé photo-activable sur l'actif cométique d'une part et sur la matière kératinique d'autre part peut se faire en une seule étape, mais il est aussi possible, si cette procédure présente des avantages, de greffer préalablement le composé photo-activable sur l'actif cosmétique puis de greffer cette association sur les matières kératiniques ou encore de greffer le composé photo-activable sur les matières kératiniques puis de greffer cette association sur l'actif cosmétique.

Tous les actifs cosmétiques qui possèdent des liaisons susceptibles de donner des réactions d'insertion avec les espèces activées issues des groupes photo-activables pourront être utilisés. Ces actifs pourront éventuellement présenter plusieurs sites de réactions avec un ou plusieurs types d'espèces activées. Il s'agit de polymères naturels ou synthétiques, solubles ou insolubles dans l'eau, de particules minérales (métalliques ou non) ou organiques (latex, polystyrènes, silicones), de filtres solaires, d'anti-oxydants.

En tant qu'actif cosmétique sous forme de polymère on compte les silicones, les polymères cationiques, les polymères amphotères.

En tant qu'actif cosmétique, sous forme de particules minérales, on compte les nacres, les pigments, ou bien encore les nanopigments (taille moyenne des particules primaires: généralement entre 5 nm et 100 nm, de préférence entre 10 nm et 50 nm) d'oxydes métalliques enrobés ou non, comme par exemple des nanopigments d'oxyde de titane (amorphe ou cristallisé sous forme rutile et/ou anatase), de fer, de zinc, de zirconium ou de cérium. On compte également l'alumine et/ou le stéarate d'aluminium.

En tant qu'actif cosmétique sous forme de filtres solaires, on compte les dérivés 1,3,5-triazine, dérivés du dibenzoylméthane, les dérivés cinnamiques, les anthranilates; les dérivés salicyliques, les dérivés du camphre; les dérivés de la benzophénone ; les dérivés de β,β-diphénylacrylate, les dérivés de benzotriazole ; les dérivés de benzalmalonate ; les dérivés de benzimidazole ; les imadazolines ; les dérivés bis-benzoazolyle tels que décrits dans les brevets EP 669323 et US2,463,264 ; les dérivés de l'acide p-aminobenzoique (PABA) ; les dérivés de méthylène bis-hydroxyphényl benzotriazole tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'a-alkylstyrène tels que ceux décrits dans la demande de brevet DE19855649, les 4,4-diarylbutadiènes tels que ceux décrits dans les demandes de brevet EP0967200 et DE19755649. En tant qu'agent cosmétique, on compte également les corps gras, les adoucissants, les antioxydants, les agents anti-radicaux libres, les émollients, les α-hydroxyacides, les agents hydratants, les vitamines, les agents répulsifs contre les insectes, les parfums, les anti-inflammatoires, les antagonistes de substance P, les charges, les colorants.

Les compositions selon l'invention peuvent également contenir d'autres constituants qui n'interagissent pas forcément avec les composés photo-activables.

Ces compositions peuvent également contenir des activateurs des composés photo-activables comme les polyamines.

Les compositions selon la présente invention comprennent généralement de 0,0001% à 50%, de préférence de 0,001% à 30%, et de manière encore plus préférée de 0,01% à 10% en poids de composé photo-activables par rapport au poids total de la composition.

Lorsque ces compositions comprennent au moins un autre constituant, celui-ci est présent en une quantité comprise entre 0,01 et 70%, de préférence de 1% à 50% en poids par rapport au poids total de la composition.

La présente invention concerne encore un procédé de traitement cosmétique qui consiste à appliquer une composition selon l'invention sur les matières kératiniques et à exposer les matières kératiniques à un rayonnement d'une ou plusieurs longueurs d'ondes comprises entre 200 et 450 nm, de préférence 200 et 400 nm et de manière encore plus préférée 250 et 400 nm. Ce procédé peut être mis en oeuvre en une ou plusieurs étapes.

Un procédé de traitement cosmétique spécialement intéressant consiste à combiner le procédé de traitement cosmétique selon l'invention avec un traitement classique.

## Revendications

1. Composition cosmétique contenant, dans un solvant cosmétiquement acceptable
- au moins un actif cosmétique choisi dans le groupe formé par les polymères, les particules minérale ou organiques, les filtres solaires, les anti-oxydants, les colorants, les corps gras, les adoucissants, les agents anti-raditaux libres, les émollients, les α-hydroxyacides, les agents hydratants, les vitamines, les agents répulsifs contre les insectes, les parfums, les anti-inflammatoires, les antagonistes de substance P, les charges et,
- au moins un composé photo-activable le composé photo-activable et l'actif cosmétique étant liés de manière covalente, ledit composé photo activable, comprenant une molécule-support choisie parmi (i)les chaînes linéaires ou ramifiées, contenant de 1 à 80 atomes de carbone, comprenant de manière éventuelle 1 à 25 héteroatomes, cette chaîne étant éventuellement substituée par 1 à 60 substituants choisis parmi les groupes hydroxyde, amine, thiol, carbamate, éther, acide, ester, amide, cyano, uréido, halogène ; (ii); les polymères naturels éventuellement modifiés, (iii) les polymères synthétiques ;
et sur laquelle sont liés de façon covalente au moins deux entités ou groupes fonctionnels photo-activables contenant des groupes chimiques qui, après irradiation entre 200 et 450 nm, sont transformés en espèces réactives capables de réagir avec des groupes chimiques appartenant à d'autres molécules, ces groupes fonctionnels photo-activables étant obtenus par greffage sur la molécule-support de composés choisis parmi
■ les diazirines de formule : 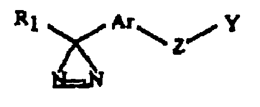 dans laquelle R₁ est choisi dans le groupe formé par un atome d'hydrogène, un radical alkyle en C₁ à C₁₀ linéaire ou ramifié, un radical, alkényle en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', CeN, COOH, F, Cl, Br, I, OR, COOR', SO₃H, COR', SH, SR', OH, où R' est un radical alkyle en C₁ à C₁₀.
Z est une simple liaison ou un groupe espaceur qui est une chaîne carbonée, linéaire ramifiée ou cyclique, saturée ou insaturée, en C₁-C₁₀₀, de préférence C₁-C₅₀, cette chaîne pouvant être interrompue par des hétéroatomes tels que le soufre, l'oxygène, l'azole, le silicium ou le phosphore et pouvant aussi comprendre un ou plusieurs substituants tels que des groupes hydroxyles, amines, thiols, carbamates, éthers, acides, esters, amides, cyano, uréido.
Y représente une fonction choisie dans le groupe formé par les alcools, amines, thiols, thiosulfates, acides carboxyliques et ses dérivés tels que les anhydrides, chlorures d'acide, esters, les acétals et hémi-acétals, les aminals et hémi-aminals, les cétones, les aldéhydes, alpha-hydroxycétones, les alpha-halocétones époxydes, les lactones, thiolactones, azalactones, isocyanate, thiocyanate, imines, imides (succinimides, glutimides), imidoesters, aziridines, imidates, oxazine et oxazoline, oxazinium et oxazolinium, les halogénes (fluor, chlore, iode, brome), les chlorotriazines, chloropyrimidines, chloroquinoxalines, chlorobenzotriazoles, les halogénures (X= F, Cl, I ou Br) de sulfonyle : SO₂X, les siloxanes, silanols, silanes, les pyridyldithio, les N-hydroxysuccinimide esters, les vinyles activés ou non activés parmi lesquels les acrylonitriles, esters acryliques et méthacryliques, acides et esters crotoniques, acides et esters cinnamiques, styrènes, butadiènes, éthers de vinyle, vinyle cétonc, esters maléiques, maléimides, vinyl sulfones, les hydrazines, les phenyls glyoxals.
Ar représente un noyau aromatique choisi dans le groupe formé par : 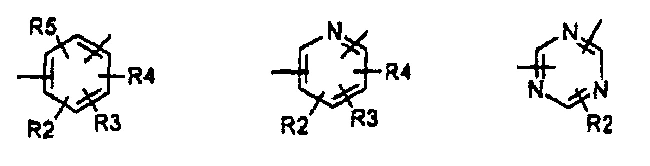 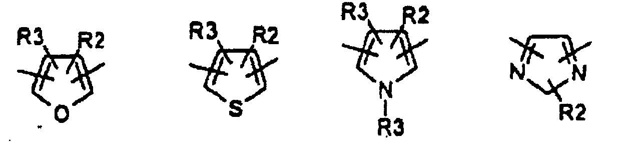 dans lesquels R₂, R₃, R₄, et R₅ représentent, indépendamment l'un de l'autre, des radicaux choisis dans le groupe formé par un atome d'hydrogène, un radical alkyle en C₁ à C₁₀ linéaire ou ramifié, un radical alkényle en C₂ à C₁₀ linéaire ou ramifié, un alkynyle en C₂ à C₁₀ linéaire ou ramifié, CF₃, CCl₃, CBr₃, NR'₃+, SR'₂+, SH₂+, NH₃+, NO₂, SO₂R', C≡N, COOH, F, Cl, Br, I, OR', COOR', COR', SH, SR', OH, SO₃H où R' est un radical alkyle en C₁ à C₁₀,

2. Composition selon la revendication 1 tel que la molécule-support est un polymère naturel choisi parmi les protéines, les polysaccharides.

3. Composition selon la revendication 2 tel que la molécule-support est choisie parmi l'albumine, l'ovalbumine, la kératine, le collagène.

4. Composition selon la revendication 1 tel que la molécule-support est un polymère de synthèse choisi parmi les dendrimères, les polymères hyperramifiés, les dendrons.

5. Utilisation de la composition cosmétique selon l'une des revendications 1 à 4 en tant qu'agent de coloration, en tant qu'agent hydratant, en tant qu'agent destiné à augmenter la brillance notamment des cheveux (agent brillant), en tant que filtre scolaire, en tant qu'agent de conditionnement, en tant qu'agent de mise en forme des fibres kératiniques.

6. Procédé de traitement cosmétique **caractérisé en ce qu'**il consiste à appliquer sur les matières kératiniques une composition cosmétique selon l'une des revendications 1 à 4 et à exposer lesdites matières kératiniques à un rayonnement d'une ou plusieurs longueurs d'ondes comprises entre 200 et 450 nm, de préférence 200 et 400 nm et de manière encore plus préférée 250 et 400 nm.

7. Procédé selon la revendication 6 tel qu'il s'agit d'un procédé en une étape.

8. Procédé selon la revendication 6 tel qu'il consiste à appliquer un ajout supplémentaire d'une quantité de composé photo-activable sur l'actif cosmétique déjà greffé.

## Patentansprüche

1. Kosmetikzusammensetzung, die in einem kosmetisch unbedenklichen Lösungsmittel Folgendes enthält:
- mindestens einen kosmetischen Wirkstoff, ausgewählt aus der Gruppe der Polymere, mineralischen oder organischen Partikel, Sonnenschutzfilter, Antioxidantien, Farbstoffe, Fettkörper, Weichmacher, Radikalfänger, Emollientien, α-Hydroxysäuren, Feuchtigkeitsspender, Vitamine, Insektenabwehrmittel, Duftstoffe, entzündungshemmenden Mittel, Substanz-P-Antagonisten, Ladungen, und
- mindestens eine photoaktivierbare Verbindung, wobei die photoaktivierbare Verbindung und der Kosmetikwirkstoff kovalent gebunden sind, wobei die photoaktivierbare Verbindung ein Trägermolekül ausgewählt aus der Reihe (i) gerade oder verzweigte Ketten, die 1 bis 80 Kohlenstoffatome enthalten und die gegebenenfalls 1 bis 25 Heteroatome enthalten, wobei die Kette gegebenenfalls durch 1 bis 60 Substituenten, ausgewählt aus der Reihe Hydroxyl- Amin-, Thiol-, Carbamat-, Ether-, Säure-, Ester-, Amid-, Cyano-, Ureido-, Halogengruppen substituiert ist; (ii) gegebenenfalls modifizierte natürliche Polymere, (iii) synthetische Polymere umfasst;
und auf der mindestens zwei photoaktivierbare funktionelle Einheiten oder Gruppen kovalent gebunden sind, die chemische Gruppen enthalten, die nach Bestrahlung bei zwischen 200 und 450 nm in Chlorchinoxaline, Chlorbenzotriazole, Sulfonylhalogenide (X= F, Cl, I oder Br): SO₂X, Siloxane, Silanole, Silane, Pyridyldithio, N-Hydroxysuccinimidester, gegebenenfalls aktivierte Vinyle, darunter Acrylnitrile, Acrylsäureester und Methacrylsäureester, Crotonsäure und Crotonsäureester, Zimtsäure und Zimtsäureester, Styrole, Butadiene, Vinylether, Vinylketon, Maleinsäureester, Maleimide, Vinylsulfone, Hydrazine, Phenylglyoxale bedeutet,
Ar einen aromatischen Ring bedeutet, der aus der folgenden Gruppe ausgewählt ist: 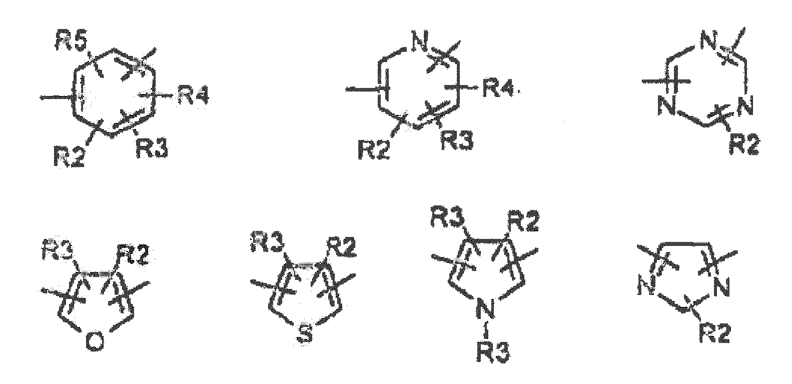 in denen R₂, R₃, R₄ und R₅ unabhängig voneinander Reste bedeuten, die aus der Gruppe Wasserstoffatom, geradkettiger oder verzweigter C₁ bis C₁₀-Alkylrest, geradkettiger oder verzweigter C₂ bis C₁₀-Alkenylrest, geradkettiger oder verzweigter C₂ bis C₁₀-Alkinylrest, CF₃, CCl₃, CBr₃, NR'₃+, SR'₂+, SH₂+, NH₃+, NO₂, SO₂R', C=N, COOH, F, Cl, Br, I, OR', COOR', COR', SH, SR', OH, SO₃H ausgewählt sind, wobei R' einen C₁ bis C₁₀-alkylrest bedeutet.

2. Zusammensetzung nach Anspruch 1, in der es sich bei dem Molekülträger um ein natürliches Polymer ausgewählt aus der Reihe Proteine und Polysaccharide handelt.

3. Zusammensetzung nach Anspruch 2, in der der Molekülträger aus der Reihe Albumin, Ovalbumin, Keratin und Collagen ausgewählt ist.

4. Zusammensetzung nach Anspruch 1, in der es sich bei dem Molekülträger um ein synthetisches Polymer ausgewählt aus der Reihe Dendrimere, hyperverzweigte Polymere und Dendrone handelt.

5. Verwendung der Kosmetikzusammensetzung nach einem der Ansprüche 1 bis 4 als Färbemittel, als feuchtigkeitspendendes Mittel, als glanzverstärkendes Mittel, insbesondere für das Haar (Glanzmittel), als Sonnenschutzfilter, als Pflegespülungsmittel, als Formgebungsmittel für Keratinfasern.

6. Kosmetisches Behandlungsverfahren, **dadurch gekennzeichnet, dass** man eine Kosmetikzusammensetzung nach einem der Ansprüche 1 bis 4 auf Keratinsubstanzen aufträgt und die Keratinsubstanzen mit einer oder mehreren Wellenlängen zwischen 200 und 450 nm, vorzugsweise 200 und 400 nm und noch stärker bevorzugt 250 und 400 nm bestrahlt.

7. Verfahren nach Anspruch 6, bei dem es sich um ein einstufiges Verfahren handelt.

8. Verfahren nach Anspruch 6, das darin besteht, dass man eine hilfsmäßige Zugabe einer Menge einer photoaktivierbaren Verbindung auf den bereits gepfropften Kosmetikwirkstoff aufträgt.

## Claims

1. Cosmetic composition containing, in a cosmetically acceptable solvent,
- at least one cosmetic active agent chosen from the group formed by polymers, mineral or organic particles, sunscreens, antioxidants, colorants, fatty substances, softeners, free-radical scavengers, emollients, α-hydroxy acids, moisturizers, vitamins, insect repellants, fragrances, antiinflammatory agents, substance P antagonists and fillers, and
- at least one photoactivatable compound, the photoactivatable compound and the cosmetic active agent being covalently bonded, the said photoactivatable compound comprising a support molecule chosen from (i) linear or branched chains containing from 1 to 80 carbon atoms, optionally comprising 1 to 25 heteroatoms, this chain optionally being substituted with 1 to 60 substituents chosen from hydroxyl, amine, thiol, carbamate, ether, acid, ester, amide, cyano, ureido and halogen groups; (ii) optionally modified natural polymers, (iii) synthetic polymers;
and to which are covalently bonded at least two photoactivatable functional species or groups containing chemical groups which, after irradiation between 200 and 450 nm, are converted into reactive species capable of reacting with chemical groups belonging to other molecules, these photoactivatable functional groups being obtained by grafting onto the support molecule compounds chosen from
▪ diazirines of formula : 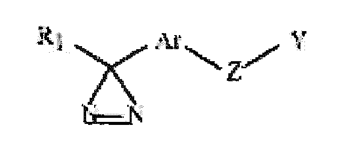 in which R₁ is chosen from the group formed by a hydrogen atom, a linear or branched C₁ to C₁₀ alkyl radical, a linear or branched C₂ to C₁₀ alkenyl radical, a linear or branched C₂ to C₁₀ alkynyl, CF₃, CCl₃ CBr₃, NR'₃+, SR'₂+, SH₂+, NH₃+, NO₂, SO₂R', C≡N, COOH, F, Cl, Br, I, OR, COOR', SO₃H, COR', SH, SR' and OH, in which R' is a C₂ to C₁₀ alkyl radical,
Z is a single bond or a spacer group which is a linear or branched or cyclic, saturated or unsaturated C₁-C₁₀₀ and preferably C₁-C₅₀ carbon-based chain, this chain possibly being interrupted with heteroatoms such as sulfur, oxygen, nitrogen, silicon or phosphorus, and also possibly comprising one or more substituents such as hydroxyl, amine, thiol, carbamate, ether, acid, ester, amide, cyano or ureido groups,
Y represents a function chosen from the group formed by alcohols, amines, thiols, thiosulfates, carboxylic acids and derivatives thereof such as anhydrides, acid chlorides, esters, acetals and hemiacetals, aminals and hemiaminals, ketones, aldehydes, α-hydroxy ketones, α-halo ketones, epoxides, lactones, thiolactones, azalactones, isocyanate, thiocyanate, imines, imides (succinimides or glutimides), imido esters, aziridines, imidates, oxazine and oxazoline, oxazinium and oxazolinium, halogens (fluorine, chlorine, iodine or bromine), chlorotriazines, chloropyrimidines, chloroquinoxalines, chlorobenzotriazoles, sulfonyl halides (X = F, Cl, I or Br): SO₂X, siloxanes, silanols, silanes, pyridyldithio derivatives, N-hydroxysuccinimide esters, activated or nonactivated vinyls including acrylonitriles, acrylic esters and methacrylic esters, crotonic acids and esters, cinnamic acids and esters, styrenes, butadienes, vinyl ethers, vinyl ketone, maleic esters, maleimides, vinyl sulfones, hydrazines and phenyl glyoxals,
Ar represents an aromatic nucleus chosen from the group formed by: 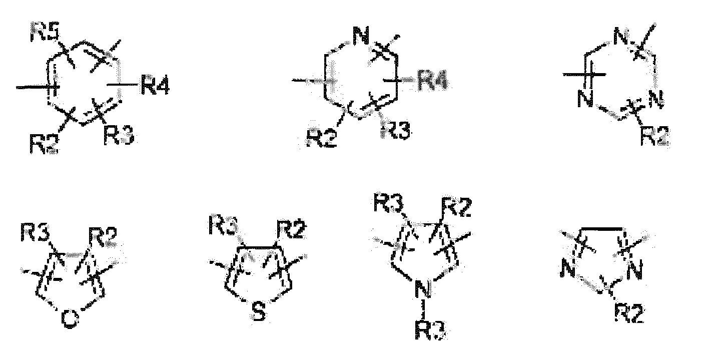 in which R₂, R₃, R₄ and R₅ represent, independently of each other, radicals chosen from the group formed by a hydrogen atom, a linear or branched C₁ to C₁₀ alkyl radical, a linear or branched C₂ to C₁₀ alkenyl radical, a linear or branched C₂ to C₁₀ alkynyl, CF₃, CCl₃, CBr₃, NR'₃+, SR'₂+, SH₂+, NH₃+, NO₂, SO₂R', C=N, COOH, F, Cl, Br, I, OR', COOR', COR', SH, SR', OH or SO₃H, in which R' is a C₁ to C₁₀ alkyl radical.

2. Composition according to Claim 1, such that the support molecule is a natural polymer chosen from proteins and polysaccharides.

3. Composition according to Claim 2, such that the support molecule is chosen from albumin, ovalbumin, keratin and collagen.

4. Composition according to Claim 1, such that the support molecule is a synthetic polymer chosen from dendrimers, hyperbranched polymers and dendrons.

5. Use of the cosmetic composition according to one of Claims 1 to 4, as a coloring agent, as a moisturizer, as an agent for increasing the sheen, especially of the hair (sheen agent), as a sunscreen, as a conditioner or as an agent for shaping keratin fibers.

6. Cosmetic treatment process, **characterized in that** it consists in applying to keratin materials a cosmetic composition according to one of Claims 1 to 4 and in exposing said keratin materials to a radiation of one or more wavelengths of between 200 et 450 nm, preferably 200 and 400 nm and even more preferably 250 and 400 nm.

7. Process according to Claim 6, such that it is a one-step process.

8. Process according to Claim 6, such that it consists in applying a further addition of an amount of photoactivatable compound to the already-grafted cosmetic active agent.
